# EUROPEAN PATENT SPECIFICATION

(11) **EP 4 533 072 B1**
(45) Date of publication and mention of the grant of the patent: **27.05.2026**
(21) Application number: 23730062.9
(22) Date of filing: 30.05.2023
(51) Int. Cl.: G01N 22/04, G01N 33/02

(54) **METHOD FOR DETERMINING AN INTERNAL QUALITY OF A FRUIT**
VERFAHREN ZUR BESTIMMUNG EINER INNEREN QUALITÄT EINER FRUCHT
PROCÉDÉ DE DÉTERMINATION DE LA QUALITÉ INTERNE D'UN FRUIT

(30) Priority: 01.06.2022 NL 2032045
(43) Date of publication of application: 09.04.2025
(73) Proprietor: Vertigo Technologies B.V., 2629 JD Delft (NL)
(72) Inventor: GALATRO, Luca, 2629 JD DELFT (NL); ROMANO, Raffaele, 2629 JD DELFT (NL); DE MARTINO, Carmine, 2629 JD DELFT (NL)
(74) Representative: EP&C
(86) International application number: PCT/EP2023/064451
(87) International publication number: WO 2023/232817

(56) References cited:
- CN-A- 103 901 049
- JP-A- S62 123 340
- SU-A1- 1 529 087
- US-B1- 6 204 670

## Description

The invention relates to a method for measuring an internal quality of a fruit wherein use is made of a microwave measuring system. The invention further relates to a microwave measuring system for measuring an internal quality of a fruit.

Internal quality of fruit, e.g. a fresh fruit, is considered a main factor that affects a decision of consumers to repurchase a specific fruit. For this reason, a lot of effort is spent in the entire fresh fruit value chain in order to make sure that fruits do not present any major internal defects, and can arrive to the distribution stage with the right set of features, such as ripening stage and flavour, while also being able to maintain an adequate shelf life.

A way of properly assessing the internal quality of fresh fruit is to cut the fruit and use several invasive techniques to identify the specific attributes of interest. This implies that the fruits on which the quality control is performed cannot be used anymore for distribution and, consequently, that only a small number of fruits can be inspected. For these reasons, the typical procedures of quality assessment of fruits are inefficient and intrinsically wasteful.

A major part of waste in the supply chain in the post-harvest phase, and before retail distribution, is related to the way decisions are made based on destructive measurements. It is customary, for example, to discard entire batches of fruit, often including thousands pieces of fruit, if just one or two pieces present major internal defects, out of a total of ten to twenty inspected pieces from the entire batch.

A way to address the above problem comes from the availability of non-invasive sensing techniques, that allow measuring internal quality of fruit without cutting. Several sensing approaches are known in the art.

In one approach, pressure sensors, such as durometers, are used to non-invasively assess the firmness of fruits, which may be related to a ripening stage of the fruit.

In other approaches, multiple fruit parameters may be assessed simultaneously. For example, optical based techniques, such as multispectral or hyperspectral imaging, as well as near-infrared spectroscopy (NIR) have been used. These techniques are based on the use of electromagnetic radiation at short wavelengths, e.g. between 400 nm and 2500 nm, that is presented to the fruit and measuring the reflected or refracted light through the fruit. These techniques are widely used due to the relatively simple technology behind them, and relative small costs associated. Nonetheless, while these technologies can present some correlation in the prediction of internal quality attributes, they may obtain inaccurate data or information related to the internal quality of the fruit, e.g. for fruits with a relatively thick exocarp such as melons, avocados, mangos, citrus fruit, apples or pears. These limitations are related to the small wavelength used, which does not allow radiation to properly penetrate into the fruit before being dissipated.

An alternative to techniques using short wavelengths, is the use of techniques based on microwave radiation, e.g. such as techniques based on microwave spectroscopy, where wavelengths are longer than 1 cm. These techniques allow higher penetration in the material due to the longer wavelengths of the microwaves penetrating deeper into the material.

An example of a measuring technique based on microwave radiation is disclosed in WO03091715, wherein a sensing apparatus is disclosed for sensing properties of a non-conductive object. The sensing apparatus includes a microwave source to generate microwave signals, a transmitting antenna, a receiving antenna, and a microwave reflectometer. A processor determines a measure of a property of the object based on a transmission coefficient of the signal represented by scattering parameters.

Another example of a measuring technique based on microwave radiation is disclosed in CN103901049, wherein a measuring device is disclosed that is based on the use of a transceiver probe that is placed on the surface of a fruit, wherein the probe emits a microwave signal directly on the surface of the fruit for measuring the fruit properties.

SU1529087 discloses a known method and apparatus for determining the internal quality of a fruit using a microwave measuring system whereby microwave frequency oscillations are directed from a generator through a coupler and an antenna onto a fruit to be tested.

It is an object of the invention to provide an alternative method of determining an internal quality of fruit. It is a further object of the invention to provide an improved method for determining an internal quality of fruit having a higher dynamic range .

The object of the invention is achieved by the method for determining an internal quality of fruit of claim 1 and a corresponding system according to claim 8.

The method of the invention is an improved method because it allows to determine an internal quality of a fruit with a higher dynamic range. The method relies on measuring a complex reflection coefficient, and using this reflection coefficient as input in a regression model, within the Fraunhofer distance of the antenna. By emitting and receiving the microwave signal with the same antenna in the Fraunhofer distance, the microwave field is contained, e.g. the large majority of the entire field impacts the fruit and reflects back towards the antenna, between the fruit and the antenna. Thus little electromagnetic power is lost and a higher dynamic range is achieved. Determining the internal quality of the fruit based on, e.g., a transmission coefficient implies an increase in lost electromagnetic power because the signal has to travel between two antennas and through the fruit. A further advantage of the method of the invention is that there is no need for using focusing strategies, e.g. using focal lenses and more complex setups, and only a single antenna is required, thus a simpler and cheaper setup is achieved.

The method of the invention relies on placing or providing the microwave antenna within the Fraunhofer distance of the antenna from the fruit. Thus the antenna and fruit are placed in the near field regime relative to each other. The Fraunhofer distance is defined as dF = 2D²/λ, where D is the linear dimension of the antenna and λ is the wavelength of the emitted microwave through the medium and depends on the relative permittivity of the material in which the wave is propagating.

In some embodiments of the invention the method further comprises:
- determining the Fraunhofer distance of the antenna for the microwave frequency range using the formula dF = 2D²/λ, wherein dF is the Fraunhofer distance, D is the linear dimension of the antenna and λ is the wavelength of the emitted microwave, depending on the permittivity of the material through which the microwaves radiate.

For example, the Fraunhofer distance may be determined when calibrating the regression model on the processor.

Methods relying on closed-form calculations, e.g. methods relying on determining the dielectric permittivity, intrinsically are less reliable in the near-field regime compared to the far-field regime, because these methods implicitly assume plane-wave propagation of the microwaves, e.g. it is assumed in determining the dielectric permittivity from the measured signal that the fruit is located at a distance larger than the Fraunhofer distance from the antenna. For example, in this case the dielectric permittivity is determined through a closed-form extraction from the scattering parameters.

In order to allow determining of an internal quality of the fruit while the antenna is placed in the Fraunhofer distance, the method of the invention relies on a regression model relating the complex reflection coefficient, e.g. the phase, amplitude, real part, imaginary part and derivatives thereof, e.g. over the microwave frequency range, to one or more quality attributes of the fruit. The regression model may be determined by a calibration procedure wherein, for example, the complex reflection coefficient of a fruit is determined and, subsequently, the quality attributes of the fruit are measured in an independent way. By repeating this process, the regression model may be determined experimentally. The regression model may be trained based on principal component methods, that allow to minimize the number of features employed during the training.

The internal quality of the fruit may be a ripening stage, shelf life, internal defects and flavour of the fruit. For example, the internal quality of the fruit may be indicated by a grade, A, B, C, ... , which is determined based on the ripening stage, shelf life and flavour of the fruit. Further, the internal quality of the fruit may also be related to internal defects, such as browning, greying, or hollows, of the fruit which may relate to fitness for consumption. The internal quality of the fruit is related to quality attributes of the fruit such as dry matter, brix, firmness, acidity, juiciness, internal rotting and internal de-coloration. These quality attributes may be related to the complex reflection coefficient through the regression model.

The microwave system used for determining the internal quality of the fruit comprises a microwave antenna for emitting and receiving microwave signals in a microwave frequency range. The microwave antenna may be a single microwave antenna or a microwave antenna array. The antenna is configured to be placed at the non-zero measuring distance which is smaller than the Fraunhofer distance of the antenna to the fruit, e.g. depending on the medium through which the wave propagates, when emitting microwaves. The antenna is further configured to emit a microwave signal in the microwave frequency range towards the fruit when the antenna is placed at the measuring from the fruit and to receive a reflected microwave from the fruit. The reflected microwave results from the emitted microwave being reflected by the fruit and is thus, indirectly, sourced by the antenna. The microwave frequency range may be in a frequency range between 0.1 GHz and 30 GHz. The microwave frequency range may be various subranges between 0.1 GHz and 30 GHz. Such that the emitted microwave may be a microwave band over the frequency range and the reflected microwave may be a microwave band over the frequency range. As a result, the complex reflection coefficient, e.g. a phase, amplitude, real part, imaginary part and derivatives thereof, may be a function over the microwave frequency range, or subsets thereof. The antenna may emit a plurality of discrete microwave signals over the microwave frequency range to the fruit, wherein each signal has a different frequency. In other embodiments the microwave measuring system may make use of a pulsed based VNA, which allows the antenna to emit short pulses in the time domain that, when transformed to the frequency domain, would cover a frequency range containing the frequency range for the measurements.

The measuring system further comprises a microwave source which is connected to the microwave antenna for generating the emitted microwave signal in the microwave frequency range. The microwave source may be any suitable microwave source, e.g. a broadband microwave source. The measuring system may in embodiments comprise a reflectometer stage, connected to the antenna, for separating the emitted and reflected wave. The reflectometer and microwave source may be embodied as a vector network analyser (VNA).

The measuring system also comprises a processor that is connected to the microwave antenna, and preferably to the microwave source, wherein the processor comprises a regression model, preferably determined by a calibration procedure, directly correlating a complex reflection coefficient of the fruit with one or more quality attributes of the fruit. The regression model may relate the phase, amplitude, real part, imaginary part and derivatives of the complex reflection coefficient to the one or more quality attributes. The processor may be connected to the microwave source, such that the microwave source may cause a microwave to be emitted when instructed by the processor.

The method comprises the steps of:
- placing the microwave antenna at the measuring distance from the surface of the fruit;
- emitting the microwave signal in the microwave frequency range from the microwave antenna towards the fruit;
- receiving the reflected microwave signal with the antenna;
- determining the complex reflection coefficient of the fruit over the microwave frequency range by comparing the emitted microwave signal to the reflected microwave signal;
- directly determining, by the programming on the processor using the regression model with the determined complex reflection coefficient over the microwave frequency range as input, the one or more quality attributes of the fruit;
- determining, by the programming on the processor, the internal quality of the fruit based on the quality attributes, e.g. by comparing the quality attributes to one or more threshold values and determining the internal quality depending if the quality attributes are higher or lower than the threshold values; and
- outputting the determined internal quality of the fruit.

In embodiments, the microwave measuring system further comprises a non-conductive layer provided on the microwave antenna, wherein the non-conductive layer has a thickness smaller than the Fraunhofer distance of the microwave antenna through the non-conductive layer to the fruit, and wherein the method comprises:
- placing the non-conductive layer on the surface of the fruit so that the non-conductive layer is touching the fruit and the antenna is at the measuring distance smaller than the Fraunhofer distance through the non-conductive layer from the fruit.

In this embodiment, the Fraunhofer distance and thus the maximum thickness of the non-conductive layer, have to be determined based on the permittivity of the non-conductive layer. This embodiment has various advantages. This embodiment allows to use the microwave measuring system as a handheld device, wherein the non-conductive layer allows the antenna to be positioned at the correct distance from the fruit. Another advantage is that, because the non-conductive layer is placed in contact with the fruit, the distance between the antenna and the fruit is always the same, which reduces possible measurement errors due to deviations, e.g. as a result of dispersion of the microwave signal, in distance between the antenna and the fruit, which may be advantages when measuring fruits of different sizes. This embodiment differs from known measuring systems using a probe to measure fruit qualities because in the known systems the antenna in the probe is in direct contact with the fruit, whereas in this embodiment the antenna is not in direct contact with the fruit.

In embodiments, the method further comprises:
- performing a time-domain transformation to the complex reflection coefficient or the emitted and reflected microwaves to determine the measuring distance between the fruit and the microwave antenna; and
- using the determined measuring distance as input in the regression model for determining the quality attributes.

In these embodiments, the actual measuring distance, which may vary, e.g. due to various fruit sizes, is determined based on the time-domain transformed complex reflection coefficient or based on the emitted and reflected microwaves.

Small deviations in the measuring distance, e.g. as a result of difference in fruit sizes, may have an impact on the determining of the quality attributes of the fruit through variations in the measured reflection coefficient. In this embodiment, the measuring distance is determined by performing a time-domain transformation on the complex reflection coefficient, or equivalently on the emitted and reflected microwaves, to be able to determine the measuring distance for a specific fruit. The determined measuring distance is than used as an additional input in the regression model for determining the quality attributes of the fruit. Performing a time-domain transformation to determine the measuring distance may also be used to calibrate the measuring system, e.g. the regression model. In other embodiments, the time-domain transformed complex reflection coefficient may also be used to determine information on the dimension of the fruit, e.g. based on information on the distance between the interfaces air/fruit and fruit/air, or to determine other fruit properties relating to absorption, dispersion, and obstacles, e.g. seeds or holes, in the fruit. The time-domain transformed complex reflection coefficient may also be used in the regression model to determine these properties of the fruit, e.g. and then be used to determine the fruit quality.

The complex reflection coefficient may have both a contribution from a reactive field of the reflected microwave and a contribution of the radiative field of the reflected microwave. The field strength of the reactive field of an antenna is proportional to one over the cube of the distance from the antenna and thus drops faster than the field strength of the radiative field which is proportional to one over the square of the distance. The antenna and the total field strength are such that the reactive field may have a, e.g. equal or similar in magnitude, contribution to the reflection coefficient as the radiative field. This allows for less energy loss because both, radiative and reactive, components of the microwave field are used.

In embodiments, the method is used in combination with a conveyor belt which is configured to transport the fruit, wherein the method further comprises:
- transporting the fruit over the conveyor belt while emitting the microwave signal and receiving the reflected microwave signal.

In this embodiment, the microwave measuring system may be part of a fruit sorting line comprising the conveyor belt. The antenna may be placed at the measuring distance, e.g. above or below, the conveyor belt to measure the reflected microwave. In these embodiments, the internal quality of the fruit may be determined in real time as the fruit passes the antenna, and based on the determined internal quality, the fruit may be sorted downstream of the antenna in the sorting line. In an advantageous embodiment, the antenna may be integrated in the conveyor belt, e.g. using a non-conductive material, e.g. such that the non-conductive material is in contact with a fruit passing on the conveyor belt.

In embodiments, method further comprises:
- sorting the fruit based on the internal quality of the fruit, e.g. providing an internal quality threshold and sorting the fruit based on whether or not the internal quality of the fruit is higher or lower than the internal quality threshold.

The regression model may be determined by a calibration procedure, by:
- determining regression coefficients based on measurements performed on a test fruit.

The regression model may be determined by a calibration procedure using principal component methods.

In embodiments, the microwave measurement system comprises a second microwave antenna for emitting and receiving microwave signals in a microwave frequency range, wherein the microwave antenna is configured to:
- be provided at a second non-zero measuring distance from the fruit, wherein the second measuring distance lies between zero and the Fraunhofer distance of the second microwave antenna to the fruit,
- emit a second microwave signal in the microwave frequency range towards the fruit when the second antenna is provided at the second measuring distance from the fruit; and
- receive a second reflected microwave signal when the second antenna is placed at the second measuring distance from the fruit, wherein the second reflected microwave signal results from the second emitted microwave signal reflected by the fruit towards the second antenna,

wherein the microwave source is connected to the second microwave antenna for generating the second emitted microwave signal and wherein the processor is connected to the second microwave antenna and wherein the regression model is configured to directly correlate a second complex reflection coefficient of the fruit with the one or more quality attributes of the fruit,
and wherein the method further comprises:
   - placing the second microwave antenna at the second measuring distance from the fruit;
   - emitting the second microwave signal in the microwave frequency range from the second microwave antenna towards the fruit;
   - receiving the second reflected microwave signal with the second antenna;
   - determining the second complex reflection coefficient of the fruit over the microwave frequency range by comparing the second emitted microwave signal to the second reflected microwave signal;
   - directly determining, by the programming on the processor using the regression model with the determined first and second complex reflection coefficients over the microwave frequency range as input, the one or more quality attributes of the fruit; and
   - determining, by the programming on the processor, the internal quality of the fruit based on the quality attributes.

The second microwave antenna may be placed opposite the microwave antenna relative to the fruit or under an angle to the microwave antenna relative to the fruit. This embodiment allows to use to reflection coefficients of two independent measurements allowing to increase the reliability of the method. The second microwave antenna may be physically connected to the first microwave antenna or the second microwave antenna may be part of a separate device, only connected to the processor, e.g. wirelessly. The first and second microwave antennas may emit microwaves simultaneously, or one after the other in order to not interfere between themselves. Additionally, the first and second microwave may emit microwaves at different frequencies, e.g. the first microwave antenna may emit a microwave at 1 GHz and the second microwave antenna may emit a microwave at 10 GHz. This may allow to more rapidly determine a reflection coefficient over a wider frequency range.

In embodiments, the method further comprises:
- receiving by the second antenna a transmitted microwave signal from the first antenna, wherein the transmitted microwave signal results from the first microwave signal transmitted by the fruit;
- determining by the processor a complex transmission coefficient of the fruit over the microwave frequency range by comparing the first emitted microwave signal to the transmitted microwave signal; and
- using the transmission coefficient as input for the regression model.

The further use of the transmission coefficient from the first microwave antenna to the second microwave antenna allows to use additional information comprised in the transmission coefficient in determining the quality attributes of the fruit. The transmission coefficient may be obtained by comparing, e.g. the magnitude and/or phase of, the first emitted microwave to the transmitted microwave signal in the fruit.

The quality attributes may comprise one or more of a pulp firmness, a brix, an acidity, an amount of dry matter, juiciness, an amount of internal rotting, an amount of internal de-coloration, and an amount of internal browning.

The internal quality of the fruit may relate to one or more of a ripening stage of the fruit, a shelf life of the fruit, a presence of internal defects of the fruit, and a fitness to undergo procedures for artificial ripening of the fruit.

In order to illustrate the relation between quality attributes and internal quality of the fruit, two examples are given.

For example, for avocadoes the dry matter content is linked to the state of maturity (on the plant) of the fruit, and it can be used to decide the time to harvest, as well as the fitness to be ripened. In some states, avocados may need to have dry matter higher than 21% to be deemed acceptable. At the same time, the firmness may be used as metric to define the ripening stage.

In another example, for mangos the dry matter content and the brix can be linked to the state of maturity on the plant of mangos, as well as their fitness to be artificially ripened. As dry matter is representative of the starch content, and brix of the sugar content, and ripening process is responsible of conversion of starch into sugar, the capability of knowing at the same time dry matter and brix allows for a better estimate of the fitness for ripening of the measurement of dry matter alone, which is not available with other non-destructive techniques. Also here, firmness is a metric to determine the ripening stage of the fruit and decide when it is fit for consumption. Also in this case, the capability of knowing at the same time firmness and brix may be a better indicator of ripening than the firmness alone, which is typically the only parameter used for simplicity. At the same time, firmness and brix can be used to assess shelf life, while the ratio between acidity and brix allows to define metrics related to flavor and liking. Finally, the capability of assessing internal browning and other forms of internal rotting (like nose rotting) allows to determine fitness to consumption.

Outside the scope of the claims, the regression model may use the phase of the complex reflection coefficient, the magnitude of the complex reflection coefficient, the real part of the complex reflection coefficient, the imaginary part of the complex reflection coefficient, the weight of the fruit and/or the time domain transformation of the complex reflection coefficient as input for determining the one or more quality attributes of the fruit.

The invention is also related to a microwave measuring system for determining an internal quality of a fruit, comprising:
- a microwave antenna for emitting and receiving microwave signals in a microwave frequency range, wherein the microwave antenna is configured to:
   ∘ be provided at a non-zero measuring distance from the fruit, wherein the measuring distance lies between zero and the Fraunhofer distance of the microwave antenna to the fruit,
   ∘ emit a microwave signal in the microwave frequency range towards the fruit when the antenna is provided at the measuring distance from the fruit; and
   ∘ receive a reflected microwave signal when the antenna is placed at the measuring distance from the fruit, wherein the reflected microwave signal results from the emitted microwave signal reflected by the fruit towards the antenna,
- a microwave source connected to the microwave antenna for generating the emitted microwave signal in the microwave frequency range; and
- a processor connected to the microwave antenna, and preferably to the microwave source, wherein the processor comprises programming for running a regression model, preferably determined by a calibration procedure, configured to directly correlate a complex reflection coefficient of the fruit with one or more quality attributes of the fruit,
wherein the programming on the processor is configured to:
- directly determine, using the regression model with a complex reflection coefficient over the microwave frequency range as input, the one or more quality attributes of the fruit, wherein the complex reflection coefficient is determined over the microwave frequency range by comparing the emitted microwave signal to the reflected microwave signal;
- determine the internal quality of the fruit based on the quality attributes, e.g. by comparing the quality attributes to one or more threshold values and providing a quality label depending if the quality attributes are higher or lower than the threshold values; and
- outputting the determined internal quality of the fruit

In embodiments of the microwave measuring system, the system further comprises a non-conductive layer provided on the microwave antenna, wherein the non-conductive layer has a thickness smaller than the Fraunhofer distance of the microwave antenna through the non-conductive layer to the fruit, and the antenna is configured to be placed with the non-conductive layer on the fruit so that the non-conductive layer is touching the fruit and the antenna is at the measuring distance smaller than the Fraunhofer distance from the fruit.

In embodiments of the microwave measuring system, the processor is further configured to:
- perform a time-domain transformation to the complex reflection coefficient to determine the measuring distance between the fruit and the microwave antenna; and
- use the measuring distance as input in the regression model.

In the microwave measuring system the complex reflection coefficient may have both a contribution from a reactive field of the reflected microwave and a contribution of the radiative field of the reflected microwave.

In embodiments of the microwave measuring system, the measurement system further comprises a conveyor belt which is configured to transport the fruit.

In embodiments of the microwave measuring system, the processor is further configured to sort the fruit based on the internal quality of the fruit, e.g. by sorting the fruit based on whether or not the internal quality of the fruit is higher or lower than an internal quality threshold.

Outside the scope of the claims, a regression model may be determined by a calibration procedure, wherein the calibration procedure comprises:
- determining regression coefficients based on measurements performed on a test fruit.

In embodiments of the microwave measuring system, the microwave measurement system comprises a second microwave antenna for emitting and receiving microwave signals in a microwave frequency range, wherein the microwave antenna is configured to:
- be provided at a second non-zero measuring distance from the fruit, wherein the measuring distance lies between zero and the Fraunhofer distance of the second microwave antenna to the fruit,
- emit a second microwave signal in the microwave frequency range towards the fruit when the second antenna is provided at the second measuring distance from the fruit; and
- receive a second reflected microwave signal when the second antenna is provided at the second measuring distance from the fruit, wherein the second reflected microwave signal results from the second emitted microwave signal reflected by the fruit towards the second antenna,

wherein the microwave source is connected to the second microwave antenna for generating the second emitted microwave signal and wherein the processor is connected to the second microwave antenna and wherein the regression model is configured to directly correlate a second complex reflection coefficient of the fruit with the one or more quality attributes of the fruit,
and wherein the programming on the processor is further configured to:
   - directly determine, using the regression model with the determined complex reflection coefficient and a second complex reflection coefficient over the microwave frequency range as input, the one or more quality attributes of the fruit wherein the second complex reflection coefficient is determined over the microwave frequency range by
   comparing the second emitted microwave signal to the second reflected microwave signal; and
- determining the internal quality of the fruit based on the quality attributes.

In embodiments of the microwave measuring system, the processor is further configured to:
- determining a complex transmission coefficient of the fruit over the microwave frequency range by comparing the first emitted microwave signal to a transmitted microwave signal measured by the second microwave antenna; and
- use the transmission coefficient as input for the regression model.

The quality attributes may comprise one or more of a pulp firmness, a brix, an acidity, a amount of dry matter, juiciness, an amount of internal rotting, an amount of internal de-coloration, and an amount of internal browning.

The internal quality of the fruit may relate to one or more of a ripening stage of the fruit, a shelf life of the fruit, a presence of internal defects of the fruit, and a fitness to undergo procedures for artificial ripening of the fruit.

Outside the scope of the claims, the processor may be configured to use the phase of the complex reflection coefficient, the magnitude of the complex reflection coefficient, the real part of the complex reflection coefficient, the imaginary part of the complex reflection coefficient, the weight of the fruit and/or the time domain transformation of the complex reflection coefficient as input for the regression model to determine the one or more quality attributes of the fruit.

The invention will be explained below with reference to the drawing, in which:
- Fig. 1 schematically shows a diagram of the method of the invention;
- Fig. 2 schematically shows a microwave measuring system of the invention;
- Fig. 3 schematically shows an antenna according to an embodiment of the invention; and
- Fig. 4 schematically shows a first antenna and a second antenna according to an embodiment of the invention.

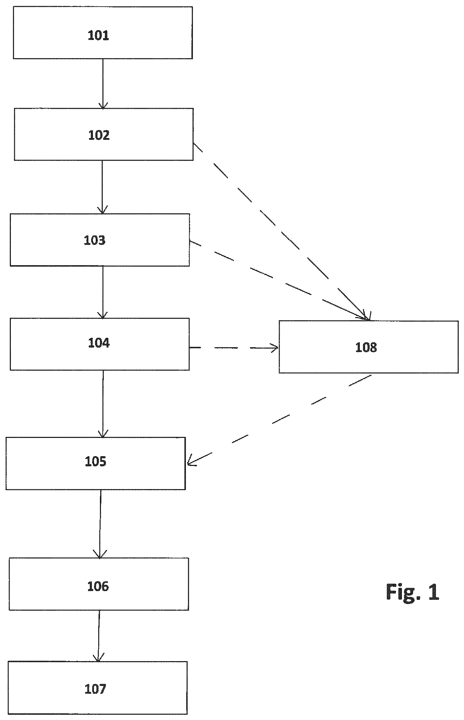

Figure 1 schematically shows a diagram of the method of the invention wherein different steps of the method are represented by different blocks in the diagram. The method comprises placing 101 the microwave antenna 3 at the measuring distance d1 from the surface of the fruit 1. The fruit 1 may be located on a measuring location, such as a conveyor belt or a storage box. For example, if the fruit 1 is provided on a conveyor belt the microwave antenna 3 may be placed at a position perpendicular to the moving direction of the conveyor belt.

Once the microwave antenna is placed at the measuring distance d1 from the surface of the fruit, the microwave signal a1 is emitted 102 in the microwave frequency range from the microwave antenna 3 towards the fruit 1. The microwave frequency range may be a subrange between 0.1 GHz and 30 GHz. For example in case the microwave measuring system 2 is a handheld system the subrange 0.5 - 14 GHz may be used. For example for a microwave measuring system 2 comprising a conveyor belt a subrange with a lower limit above 2 GHz may be used. A subrange with a lower limit above 2 GHz allows for a smaller linear dimension of the antenna 3 and therefor a narrower beamwidth, which is advantageous for measuring a moving fruit 1. Additionally, several fruit 1 on the conveyor belt may have different sizes such that the measuring distance d1 may have to be larger compared to for a handheld device, in order to allow the microwave signal to fully illuminate the fruit, a smaller beamwidth is preferable.

The emitted microwave a1 is reflected by the fruit 1 to create a reflected microwave b1, which is received 103 by the antenna 3. The antenna 3 is positioned within the Fraunhofer distance of the fruit 1 such that the emitted a1 and reflected microwaves b1 may be contained between the fruit 1 and the antenna 3 such that a substantial amount of the reflected microwave b1 is received by the antenna 3.

The method further comprises determining 104 the complex reflection coefficient of the fruit 1 over the microwave frequency range by comparing the emitted microwave signal a1 to the reflected microwave signal b1. The complex reflection coefficient may be determined by a reflectometer suitable for comparing microwave signals in the GHz range. The complex reflection coefficient may be seen as a complex function over the microwave frequency range and gives a complex number for a given frequency in the frequency range. The complex reflection coefficient is send to the processor 5. The complex reflection coefficient may depend on the emitted microwave signal a1 and the reflected microwave signal b1.

The programming on the processor directly determines 105 the one or more quality attributes of the fruit 1 using the complex reflection coefficient over the microwave frequency range as input. The regression model may be calibrated for a specific type of fruit 1 to relate the reflection coefficient to specific quality attributes suitable for the type of fruit 1. The quality attributes may be one or more of a pulp firmness, a brix, an acidity, an amount of dry matter, juiciness, an amount of internal rotting, an amount of internal de-coloration, and an amount of internal browning depending on the type of fruit. The regression model may be trained by testing a fruit using a different, e.g. invasive, method to determine the regression coefficients. The regression model may use the real part, imaginary part, phase, magnitude and derivatives thereof of the complex reflection coefficient over the frequency range as input variables.

The processor is used to determine 106 the internal quality of the fruit 1 based on the determined quality attributes. For example, by comparing the quality attributes to one or more threshold values and determining the internal quality of the fruit 1 depending on if the quality attributes are higher or lower than the threshold values. The internal quality of the fruit 1 may be used to sort the fruit 1, for example, into fruit that is suitable for consumption and fruit 1 that is not suitable for consumption.

The method further comprises outputting 107 the determined internal quality of the fruit 1, e.g. by the processor 5, e.g. to a fruit sorting device to sort the fruit 1. The determined internal quality may also be outputted on a display or as part of a list of internal qualities of measured fruits 1.

Optionally, the method comprises performing 108 a time-domain transformation on the complex reflection coefficient or the emitted a1 and reflected microwaves b1 to determine the measuring distance d1 between the fruit 1 and the microwave antenna 3. The determined measuring distance d1 is then used as input in the regression model for determining the quality attributes. The measuring distance d1 may vary between measurements, e.g. as a result of different fruit sizes or movements of the microwave antenna 3, which influences the measurement. In order to compensate this, the measuring distance d1 is determined during the measurement and used as input in the regression model which is calibrated to determine the quality attributes based on the complex reflection coefficient and the determined measuring distance d1. In this embodiment, the regression model may additionally be calibrated by measuring the measuring distance to the fruit and training the model using the measured distance as input.

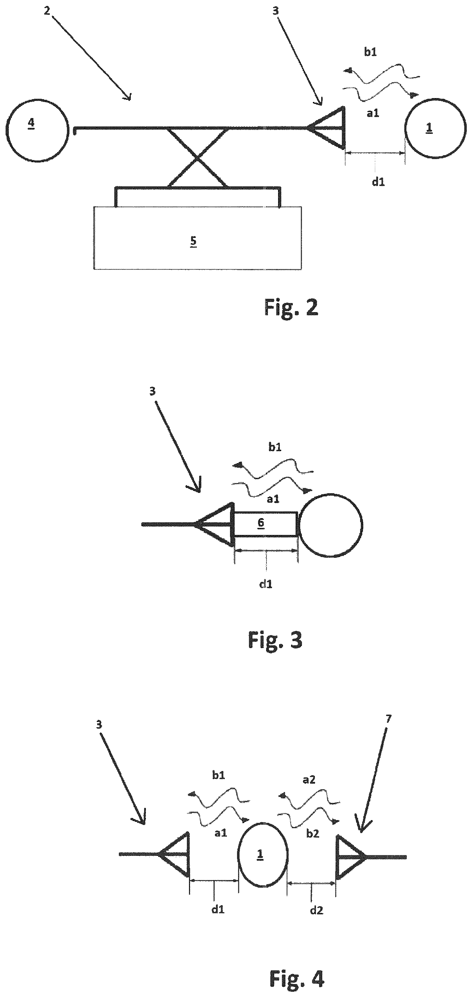

Figure 2 schematically shows a microwave measuring system 2 of the invention comprising a microwave antenna 3, a microwave source 4 and a processor 5. The microwave antenna 3 is placed at a non-zero distance d1, smaller than the Fraunhofer distance of the antenna 3, from the fruit 1 to emit emitted microwave signals a1 and receive reflected microwave signals b1. The curved arrows a1 and b1 schematically indicate the microwaves, in more real representations the microwaves should be represented directly between the antenna 3 and the fruit 1. The microwave antenna 3 may have a linear dimension, used in determining the Fraunhofer distance of the antenna 3, smaller than a size of the fruit 1.

The microwave source 4 is connected to the microwave antenna 3 for generating the emitted microwave signal a1 in the desired microwave frequency range. The microwave source 4 may be configured to generate various microwave signals, e.g. continuous microwave signals, pulsed microwave signals, discrete microwave signals, in various microwave frequency ranges as desired or required for the fruit 1 to be measured.

Figure 2 further shows a processor 5 connected to the microwave antenna 3 and the microwave source 4. The processor 5 comprises programming for running the regression model, which may be determined by a calibration procedure. The regression model is configured to directly correlate the complex reflection coefficient of the fruit 1 with one or more quality attributes of the fruit 1.

Figure 3 schematically shows an antenna 3 according to an embodiment of the invention comprising a non-conductive layer 6 which is placed on the surface of the fruit 1. For an antenna 3 comprising a non-conductive layer 6 as shown in figure 3, the Fraunhofer distance and thus the maximum thickness of the non-conductive layer6, has to be determined based on the permittivity of the non-conductive layer 6.

An antenna 3 as shown in figure 3 has various advantages. The antenna 3 allows to use the microwave measuring system 2 as a handheld device, wherein the non-conductive layer 6 allows the antenna 3 to be positioned at the correct distance from the fruit 1. Another advantage is that, because the non-conductive layer 6 is placed in contact with the fruit 1, the distance between the antenna 3 and the fruit 1 is always the same, which reduces possible measurement errors due to deviations, e.g. as a result of dispersion of the microwave signal, in distance between the antenna 3 and the fruit 1, which may be advantages when measuring fruits 1 of different sizes.

Figure 4 schematically shows a first antenna 3 and a second antenna 7 according to an embodiment of the invention. The first antenna 3 and second antenna 7 are shown opposite to each other relative to the fruit 1, however in embodiments the angle between the first antenna 3 and the second antenna 7 may vary, or be variable. The second antenna is placed at a second measurement distance d2 and configured to emit a second microwave signal a2 and receive the second reflected microwave signal b2. Either one or both of the antennas 3, 7 may comprise a non-conductive layer 6 as shown in figure 3. The fruit 1 may be placed on a conveyor belt, wherein the first antenna 3 is placed above or below the conveyor belt and the second antenna 7 is placed to a side of the conveyor belt. Depending on the angle of the first antenna 3 relative to the second antenna 7 a transmission of the first microwave signal a1 to the second antenna 7 through the fruit may be minimized to avoid possible interference from the first microwave antenna 3 in determining the second reflection coefficient.

## Claims

1. Method for determining an internal quality of a fruit (1), wherein use is made of a microwave measuring system (2) comprising:
- a microwave antenna (3) for emitting and receiving microwave signals in a microwave frequency range, wherein the microwave antenna (3) is configured to:
∘ be provided at a non-zero measuring distance (d1) from a surface of the fruit (1), wherein the measuring distance (d1) lies between zero and the Fraunhofer distance of the microwave antenna (3) to the fruit (1),
∘ emit a microwave signal (a1) in the microwave frequency range towards the fruit (1) when the antenna (3) is provided at the measuring distance (d1) from the surface of the fruit (1); and
∘ receive a reflected microwave signal (b1) when the antenna (3) is placed at the measuring distance (d1) from the fruit, wherein the reflected microwave signal (b1) results from the emitted microwave signal (a1) reflected by the fruit (1) towards the antenna (3),
- a microwave source (4) connected to the microwave antenna (3) for generating the emitted microwave signal (a1) in the microwave frequency range; and
- a processor (5) connected to the microwave antenna (3), and preferably to the microwave source (4), wherein the processor (5) comprises programming running a regression model, preferably determined by a calibration procedure, configured to directly correlate a complex reflection coefficient of the fruit (1) with one or more quality attributes of the fruit (1),
wherein the method comprises:
- placing (101) the microwave antenna (3) at the measuring distance (d1) from the surface of the fruit (1);
- emitting (102) the microwave signal (a1) in the microwave frequency range from the microwave antenna (3) towards the fruit (1);
- receiving (103) the reflected microwave signal (b1) with the antenna (3);
- determining (104) the complex reflection coefficient of the fruit (1) over the microwave frequency range by comparing the emitted microwave signal (a1) to the reflected microwave signal (b1);
- directly determining (105), by the programming on the processor (5) using the regression model with the determined complex reflection coefficient over the microwave frequency range as input, the one or more quality attributes of the fruit (1);
- determining (106), by the programming on the processor (5), the internal quality of the fruit (1) based on the quality attributes, by comparing the quality attributes to one or more threshold values and determining the internal quality depending on the quality attributes being higher or lower than the one or more threshold values; and
- outputting (107) by the processor (5) the determined internal quality of the fruit (1).

2. Method according to claim 1, wherein the microwave measuring system (2) further comprises a non-conductive layer (6) provided on the microwave antenna (3), wherein the non-conductive layer (6) has a thickness smaller than the Fraunhofer distance of the microwave antenna (3) through the non-conductive layer to the fruit (1), and wherein the method comprises:
- placing the non-conductive layer (6) on the surface of the fruit (1) so that the non-conductive layer (6) is touching the fruit (1) and the antenna (3) is at the measuring distance (d1) smaller than the Fraunhofer distance through the non-conductive layer (6) from the fruit (1).

3. Method according to one or more of the preceding claims, wherein the method further comprises:
- performing (108) a time-domain transformation on the complex reflection coefficient or the emitted (a1) and reflected microwaves (b1) to determine the measuring distance (d1) between the fruit (1) and the microwave antenna (3); and
- using the determined measuring distance (d1) as input in the regression model for determining the quality attributes.

4. Method according to one or more of the preceding claims, wherein the method is used in combination with a conveyor belt which is configured to transport the fruit (1), wherein the method further comprises:
- transporting the fruit (1) over the conveyor belt while emitting the microwave signal (a1) and receiving the reflected microwave signal (b1).

5. Method according to one or more of the preceding claims, wherein the method further comprises:
- sorting the fruit (1) based on the internal quality of the fruit (1), e.g. providing an internal quality threshold and sorting the fruit (1) based on whether or not the internal quality of the fruit (1) is higher or lower than the internal quality threshold.

6. Method according to one or more of the preceding claims, wherein the microwave measurement system (2) comprises a second microwave antenna (7) for emitting and receiving microwave signals in a microwave frequency range, wherein the second microwave antenna (7) is configured to:
- be provided at a second non-zero measuring distance (d2) from the fruit (1), wherein the second measuring distance (d2) lies between zero and the Fraunhofer distance of the second microwave antenna (7) to the fruit,
- emit a second microwave signal (a2) in the microwave frequency range towards the fruit (1) when the second antenna (7) is provided at the second measuring distance (d2) from the fruit (1); and
- receive a second reflected microwave signal (b2) when the second antenna (7) is placed at the second measuring distance (d2) from the fruit (1), wherein the second reflected microwave signal (b2) results from the second emitted microwave signal (a2) reflected by the fruit (1) towards the second antenna (7),
wherein the first microwave source (4) or a second microwave source is connected to the second microwave antenna (7) for generating the second emitted microwave signal (a2) and wherein the processor (5) is connected to the second microwave antenna (7) and wherein the regression model is configured to directly correlate a second complex reflection coefficient of the fruit (1) with the one or more quality attributes of the fruit (1),
and wherein the method further comprises:
- placing the second microwave antenna (7) at the second measuring distance (d2) from the fruit (1);
- emitting the second microwave signal (a2) in the microwave frequency range from the second microwave antenna (7) towards the fruit (1);
- receiving the second reflected microwave signal (b2) with the second antenna (7);
- determining the second complex reflection coefficient of the fruit (1) over the microwave frequency range by comparing the second emitted microwave signal (a2) to the second reflected microwave signal (b2);
- directly determining, by the programming on the processor (5) using the regression model with the determined first and second complex reflection coefficients over the microwave frequency range as input, the one or more quality attributes of the fruit (1); and
- determining, by the programming on the processor, the internal quality of the fruit (1) based on the quality attributes.

7. Method according to claim 6, wherein the method further comprises:
- receiving by the second antenna (7) a transmitted microwave signal from the first antenna (3), wherein the transmitted microwave signal results from the first microwave signal transmitted by the fruit (1);
- determining by the processor (5) a complex transmission coefficient of the fruit (1) over the microwave frequency range by comparing the first emitted microwave signal to the transmitted microwave signal; and
- using the transmission coefficient as input for the regression model.

8. Microwave measuring system (2) for determining an internal quality of a fruit (1), comprising:
- a microwave antenna (3) for emitting and receiving microwave signals in a microwave frequency range, wherein the microwave antenna (3) is configured to:
∘ be provided at a non-zero measuring distance (d1) from the fruit (1), wherein the measuring distance (d1) lies between zero and the Fraunhofer distance of the microwave antenna (3) to the fruit (1),
∘ emit a microwave signal (a1) in the microwave frequency range towards the fruit (1) when the antenna (3) is provided at the measuring distance (d1) from the fruit (1); and
∘ receive a reflected microwave signal (b1) when the antenna (3) is placed at the measuring distance (d1) from the fruit (1), wherein the reflected microwave signal (b1) results from the emitted microwave signal (a1) reflected by the fruit (1) towards the antenna (3),
- a microwave source (4) connected to the microwave antenna (3) for generating the emitted microwave signal (a1) in the microwave frequency range; and
- a processor (5) connected to the microwave antenna (3), and preferably to the microwave source (4), wherein the processor (5) comprises programming running a regression model, preferably determined by a calibration procedure, configured to directly correlate a complex reflection coefficient of the fruit (1) with one or more quality attributes of the fruit (1),
wherein the programming on the processor (5) is configured to:
- directly determine, using the regression model with a complex reflection coefficient over the microwave frequency range as input, the one or more quality attributes of the fruit (1), wherein the complex reflection coefficient is determined over the microwave frequency range by comparing the emitted microwave signal (a1) to the reflected microwave signal (b1);
- determine the internal quality of the fruit (1) based on the quality attributes, by comparing the quality attributes to one or more threshold values and providing a quality label depending on the quality attributes being erehigher or lower than the one or more threshold values; and
- outputting the determined internal quality of the fruit (1).

9. Measurement system according to claim 8, wherein the microwave measuring system (2) further comprises a non-conductive layer (6) provided on the microwave antenna (3), wherein the non-conductive layer (6) has a thickness smaller than the Fraunhofer distance of the microwave antenna (3) through the non-conductive layer (6) to the fruit (1), and the antenna (3) is configured to be placed with the non-conductive layer (6) on the fruit (1) so that the non-conductive layer (6) is touching the fruit (1) and the antenna (3) is at the measuring distance (d1) smaller than the Fraunhofer distance through the non-conductive layer to the fruit (1).

10. Measurement system according to one or more of claims 8 - 9, wherein processor (5) is further configured to:
- perform a time-domain transformation to the complex reflection coefficient or to the emitted (a1) and reflected microwaves (b1) to determine the measuring distance (d1) between the fruit (1) and the microwave antenna (3); and
- use the measuring distance (d1) as input in the regression model.

11. Measurement system according to one or more of the claims 8 - 10, wherein the measurement system (2) further comprises a conveyor belt which is configured to transport the fruit (1).

12. Measurement system according to one or more of the claims 8 - 11, wherein the processor (5) is further configured to sort the fruit (1) based on the internal quality of the fruit (1), e.g. by sorting the fruit (1) based on whether or not the internal quality of the fruit (1) is higher or lower than an internal quality threshold.

13. Measurement system according to one or more of the claims 8 - 12, wherein the microwave measurement system (2) comprises a second microwave antenna (7) for emitting and receiving microwave signals in a microwave frequency range, wherein the second microwave antenna (7) is configured to:
- be provided at a second non-zero measuring distance (d2) from the fruit (1), wherein the second measuring distance (d2) lies between zero and the Fraunhofer distance of the second microwave antenna (7) to the fruit (1),
- emit a second microwave signal (a2) in the microwave frequency range towards the fruit (1) when the second antenna (7) is provided at the second measuring distance (d2) from the fruit (1); and
- receive a second reflected microwave signal (b2) when the second antenna (7) is provided at the second measuring distance (d2) from the fruit (1), wherein the second reflected microwave signal (b2) results from the second emitted microwave signal (a2) reflected by the fruit (1) towards the second antenna (7),
wherein the microwave source (4) or a second microwave source is connected to the second microwave antenna (7) for generating the second emitted microwave signal (a2) and wherein the processor (5) is connected to the second microwave antenna (7) and wherein the regression model is configured to directly correlate a second complex reflection coefficient of the fruit (1) with the one or more quality attributes of the fruit (1),
and wherein the programming on the processor (5) is further configured to:
- directly determine, using the regression model with the determined complex reflection coefficient and a second complex reflection coefficient over the microwave frequency range as input, the one or more quality attributes of the fruit (1), wherein the second complex reflection coefficient is determined over the microwave frequency range by comparing the second emitted microwave signal (a2) to the second reflected microwave signal (b2); and
- determining the internal quality of the fruit (1) based on the quality attributes.

14. Measurement system according to claim 13, wherein the processor (5) is further configured to:
- determining a complex transmission coefficient of the fruit (1) over the microwave frequency range by comparing the first emitted microwave signal (a1) to a transmitted microwave signal measured by the second microwave antenna (7); and
- use the transmission coefficient as input for the regression model.

## Patentansprüche

1. **Verfahren** zum Bestimmen einer inneren Qualität einer Frucht (1), wobei ein Mikrowellenmesssystem (2) verwendet wird, das Folgendes umfasst:
- eine Mikrowellenantenne (3) zum Emittieren und Empfangen von Mikrowellensignalen in einem Mikrowellenfrequenzbereich, wobei die Mikrowellenantenne (3) konfiguriert ist zum:
∘ in einem Messabstand (d1) von ungleich null von einer Oberfläche der Frucht (1) bereitgestellt zu sein, wobei der Messabstand (d1) zwischen null und der Fraunhofer-Distanz der Mikrowellenantenne (3) zu der Frucht (1) liegt,
∘ Emittieren eines Mikrowellensignals (a1) im Mikrowellenfrequenzbereich zur Frucht (1) hin, wenn die Antenne (3) im Messabstand (d1) von der Oberfläche der Frucht (1) bereitgestellt ist; und
∘ Empfangen eines reflektierten Mikrowellensignals (b1), wenn die Antenne (3) in dem Messabstand (d1) von der Frucht platziert ist, wobei das reflektierte Mikrowellensignal (b1) aus dem emittierten Mikrowellensignal (a1) resultiert, das von der Frucht (1) in Richtung der Antenne (3) reflektiert wird,
- eine Mikrowellenquelle (4), die mit der Mikrowellenantenne (3) verbunden ist, um das emittierte Mikrowellensignal (a1) im Mikrowellenfrequenzbereich zu erzeugen; und
- einen Prozessor (5), der mit der Mikrowellenantenne (3) und vorzugsweise mit der Mikrowellenquelle (4) verbunden ist, wobei der Prozessor (5) eine Programmierung umfasst, die ein Regressionsmodell ausführt, das vorzugsweise durch eine Kalibrierungsprozedur bestimmt wird, die dazu konfiguriert ist, einen komplexen Reflexionskoeffizienten der Frucht (1) direkt mit einem oder mehreren Qualitätsattributen der Frucht (1) zu korrelieren,
wobei das Verfahren Folgendes umfasst:
- Platzieren (101) der Mikrowellenantenne (3) in dem Messabstand (d1) von der Oberfläche der Frucht (1);
- Emittieren (102) des Mikrowellensignals (a1) im Mikrowellenfrequenzbereich von der Mikrowellenantenne (3) zur Frucht (1);
- Empfangen (103) des reflektierten Mikrowellensignals (b1) mit der Antenne (3);
- Bestimmen (104) des komplexen Reflexionskoeffizienten der Frucht (1) über den Mikrowellenfrequenzbereich durch Vergleichen des emittierten Mikrowellensignals (a1) mit dem reflektierten Mikrowellensignal (b1);
- direktes Bestimmen (105), durch die Programmierung auf dem Prozessor (5) unter Verwendung des Regressionsmodells mit dem bestimmten komplexen Reflexionskoeffizienten über den Mikrowellenfrequenzbereich als Eingabe, des einen oder der mehreren Qualitätsattribute der Frucht (1);
- Bestimmen (106), durch die Programmierung auf dem Prozessor (5), der internen Qualität der Frucht (1) basierend auf den Qualitätsattributen, durch Vergleichen der Qualitätsattribute mit einem oder mehreren Schwellenwerten und Bestimmen der internen Qualität in Abhängigkeit davon, dass die Qualitätsattribute höher oder niedriger als der eine oder die mehreren Schwellenwerte sind; und
- Ausgeben (107), durch den Prozessor (5), der bestimmten internen Qualität der Frucht (1).

2. Verfahren nach Anspruch 1, wobei das Mikrowellenmesssystem (2) ferner eine nichtleitende Schicht (6) umfasst, die auf der Mikrowellenantenne (3) bereitgestellt ist, wobei die nichtleitende Schicht (6) eine Dicke aufweist, die kleiner als die Fraunhofer-Distanz der Mikrowellenantenne (3) durch die nichtleitende Schicht zu der Frucht (1) ist, und wobei das Verfahren Folgendes umfasst:
- Platzieren der nichtleitenden Schicht (6) auf die Oberfläche der Frucht (1), sodass die nichtleitende Schicht (6) die Frucht (1) berührt und die Antenne (3) bei dem Messabstand (d1) kleiner als die Fraunhofer-Distanz durch die nichtleitende Schicht (6) von der Frucht (1) liegt.

3. Verfahren nach einem oder mehreren der vorhergehenden Ansprüche, wobei das Verfahren ferner Folgendes umfasst:
- Durchführen (108) einer Zeitdomänentransformation an dem komplexen Reflexionskoeffizienten oder den emittierten (a1) und reflektierten Mikrowellen (b1), um den Messabstand (d1) zwischen der Frucht (1) und der Mikrowellenantenne (3) zu bestimmen; und
- Verwenden des bestimmten Messabstands (d1) als Eingabe in das Regressionsmodell zur Bestimmung der Qualitätsattribute.

4. Verfahren nach einem oder mehreren der vorhergehenden Ansprüche, wobei das Verfahren in Kombination mit einem Förderband verwendet wird, das dazu konfiguriert ist, die Frucht (1) zu transportieren, wobei das Verfahren ferner Folgendes umfasst:
- Transportieren der Frucht (1) über das Förderband, während das Mikrowellensignal (a1) emittiert und das reflektierte Mikrowellensignal (b1) empfangen wird.

5. Verfahren nach einem oder mehreren der vorhergehenden Ansprüche, wobei das Verfahren ferner Folgendes umfasst:
- Sortieren der Frucht (1) basierend auf der inneren Qualität der Frucht (1), z. B. Bereitstellen einer inneren Qualitätsschwelle, und Sortieren der Frucht (1) basierend darauf, ob die innere Qualität der Frucht (1) höher oder niedriger als die innere Qualitätsschwelle ist oder nicht.

6. Verfahren nach einem oder mehreren der vorhergehenden Ansprüche, wobei das Mikrowellenmesssystem (2) eine zweite Mikrowellenantenne (7) zum Emittieren und Empfangen von Mikrowellensignalen in einem Mikrowellenfrequenzbereich umfasst, wobei die zweite Mikrowellenantenne (7) zu Folgendem konfiguriert ist:
- in einem zweiten Messabstand (d2) von ungleich null von der Frucht (1) bereitgestellt zu sein, wobei der zweite Messabstand (d2) zwischen null und der Fraunhofer-Distanz der zweiten Mikrowellenantenne (7) zu der Frucht liegt,
- Emittieren eines zweiten Mikrowellensignals (a2) im Mikrowellenfrequenzbereich zur Frucht (1) hin, wenn die zweite Antenne (7) im zweiten Messabstand (d2) von der Frucht (1) bereitgestellt ist; und
- Empfangen eines zweiten reflektierten Mikrowellensignals (b2), wenn die zweite Antenne (7) in dem zweiten Messabstand (d2) von der Frucht (1) platziert ist, wobei das zweite reflektierte Mikrowellensignal (b2) aus dem zweiten emittierten Mikrowellensignal (a2) resultiert, das von der Frucht (1) in Richtung der zweiten Antenne (7) reflektiert wird,
wobei die erste Mikrowellenquelle (4) oder eine zweite Mikrowellenquelle mit der zweiten Mikrowellenantenne (7) zum Erzeugen des zweiten emittierten Mikrowellensignals (a2) verbunden ist, und wobei der Prozessor (5) mit der zweiten Mikrowellenantenne (7) verbunden ist, und wobei das Regressionsmodell dazu konfiguriert ist, einen zweiten komplexen Reflexionskoeffizienten der Frucht (1) direkt mit dem einen oder den mehreren Qualitätsattributen der Frucht (1) zu korrelieren,
und wobei das Verfahren ferner Folgendes umfasst:
- Platzieren der zweiten Mikrowellenantenne (7) in dem zweiten Messabstand (d2) von der Frucht (1);
- Emittieren des zweiten Mikrowellensignals (a2) im Mikrowellenfrequenzbereich von der zweiten Mikrowellenantenne (7) zur Frucht (1);
- Empfangen des zweiten reflektierten Mikrowellensignals (b2) mit der zweiten Antenne (7);
- Bestimmen des zweiten komplexen Reflexionskoeffizienten der Frucht (1) über den Mikrowellenfrequenzbereich durch Vergleichen des zweiten emittierten Mikrowellensignals (a2) mit dem zweiten reflektierten Mikrowellensignal (b2);
- direktes Bestimmen, durch die Programmierung auf dem Prozessor (5) unter Verwendung des Regressionsmodells mit dem bestimmten ersten und zweiten komplexen Reflexionskoeffizienten über den Mikrowellenfrequenzbereich als Eingabe, des einen oder der mehreren Qualitätsattribute der Frucht (1); und
- Bestimmen, durch die Programmierung auf dem Prozessor, der internen Qualität der Frucht (1) basierend auf den Qualitätsattributen.

7. Verfahren nach Anspruch 6, wobei das Verfahren ferner Folgendes umfasst:
- Empfangen, durch die zweite Antenne (7), eines transmittierten Mikrowellensignals von der ersten Antenne (3), wobei das transmittierte Mikrowellensignal aus dem ersten Mikrowellensignal resultiert, das von der Frucht (1) transmittiert wird;
- Bestimmen, durch den Prozessor (5), eines komplexen Transmissionskoeffizienten der Frucht (1) über den **Mikrowellenfrequenzbereich** durch Vergleichen des ersten emittierten Mikrowellensignals mit dem transmittierten Mikrowellensignal; und
- Verwenden des Transmissionskoeffizienten als Eingabe für das Regressionsmodell.

8. Mikrowellenmesssystem (2) zum Bestimmen einer inneren Qualität einer Frucht (1), umfassend:
- eine Mikrowellenantenne (3) zum Emittieren und Empfangen von Mikrowellensignalen in einem Mikrowellenfrequenzbereich, wobei die Mikrowellenantenne (3) konfiguriert ist zum:
∘ in einem Messabstand (d1) von ungleich null von der Frucht (1) bereitgestellt zu sein, wobei der Messabstand (d1) zwischen null und der Fraunhofer-Distanz der Mikrowellenantenne (3) zu der Frucht (1) liegt,
∘ Emittieren eines Mikrowellensignals (a1) im Mikrowellenfrequenzbereich zur Frucht (1) hin, wenn die Antenne (3) im Messabstand (d1) von der Frucht (1) bereitgestellt ist; und
∘ Empfangen eines reflektierten Mikrowellensignals (b1), wenn die Antenne (3) in dem Messabstand (d1) von der Frucht (1) platziert ist, wobei das reflektierte Mikrowellensignal (b1) aus dem emittierten Mikrowellensignal (a1) resultiert, das von der Frucht (1) in Richtung der Antenne (3) reflektiert wird,
- eine Mikrowellenquelle (4), die mit der Mikrowellenantenne (3) verbunden ist, um das emittierte Mikrowellensignal (a1) im Mikrowellenfrequenzbereich zu erzeugen; und
- einen Prozessor (5), der mit der Mikrowellenantenne (3) und vorzugsweise mit der Mikrowellenquelle (4) verbunden ist, wobei der Prozessor (5) eine Programmierung umfasst, die ein Regressionsmodell ausführt, das vorzugsweise durch eine Kalibrierungsprozedur bestimmt wird, die dazu konfiguriert ist, einen komplexen Reflexionskoeffizienten der Frucht (1) direkt mit einem oder mehreren Qualitätsattributen der Frucht (1) zu korrelieren,
wobei die Programmierung auf dem Prozessor (5) zu Folgendem konfiguriert ist:
- direktes Bestimmen, unter Verwendung des Regressionsmodells mit einem komplexen Reflexionskoeffizienten über den Mikrowellenfrequenzbereich als Eingabe, des einen oder der mehreren Qualitätsattribute der Frucht (1), wobei der komplexe Reflexionskoeffizient über den Mikrowellenfrequenzbereich durch Vergleichen des emittierten Mikrowellensignals (a1) mit dem reflektierten Mikrowellensignal (b1) bestimmt wird;
- Bestimmen der internen Qualität der Frucht (1) basierend auf den Qualitätsattributen durch Vergleichen der Qualitätsattribute mit einem oder mehreren Schwellenwerten und Bereitstellen eines Gütezeichens in Abhängigkeit davon, ob die Qualitätsattribute höher oder niedriger als der eine oder die mehreren Schwellenwerte sind; und
- Ausgeben der bestimmten internen Qualität der Frucht (1).

9. Messsystem nach Anspruch 8, wobei das Mikrowellenmesssystem (2) ferner eine nichtleitende Schicht (6) umfasst, die auf der Mikrowellenantenne (3) bereitgestellt ist, wobei die nichtleitende Schicht (6) eine Dicke aufweist, die kleiner als die Fraunhofer-Distanz der Mikrowellenantenne (3) durch die nichtleitende Schicht (6) zu der Frucht (1) ist, und die Antenne (3) dazu konfiguriert ist, mit der nichtleitenden Schicht (6) auf der Frucht (1) platziert zu werden, sodass die nichtleitende Schicht (6) die Frucht (1) berührt und die Antenne (3) bei dem Messabstand (d1) kleiner als die Fraunhofer-Distanz durch die nichtleitende Schicht zur Frucht (1) liegt.

10. Messsystem nach einem oder mehreren der Ansprüche 8 - 9, wobei der Prozessor (5) ferner zu Folgendem konfiguriert ist:
- Durchführen einer Zeitdomänentransformation an dem komplexen Reflexionskoeffizienten oder den emittierten (a1) und reflektierten Mikrowellen (b1), um den Messabstand (d1) zwischen der Frucht (1) und der Mikrowellenantenne (3) zu bestimmen; und
- Verwenden des Messabstands (d1) als Eingabe in das Regressionsmodell.

11. Messsystem nach einem oder mehreren der Ansprüche 8 - 10, wobei das Messsystem (2) ferner ein Förderband umfasst, das dazu konfiguriert ist, die Frucht (1) zu transportieren.

12. Messsystem nach einem oder mehreren der Ansprüche 8 - 11, wobei der Prozessor (5) ferner dazu konfiguriert ist, die Frucht (1) basierend auf der internen Qualität der Frucht (1) zu sortieren, z. B. durch Sortieren der Frucht (1) basierend darauf, ob die interne Qualität der Frucht (1) höher oder niedriger als eine interne Qualitätsschwelle ist oder nicht.

13. Messsystem nach einem oder mehreren der Ansprüche 8 - 12, wobei das Mikrowellenmesssystem (2) eine zweite Mikrowellenantenne (7) zum Emittieren und Empfangen von Mikrowellensignalen in einem Mikrowellenfrequenzbereich umfasst, wobei die zweite Mikrowellenantenne (7) zu Folgendem konfiguriert ist:
- in einem zweiten Messabstand (d2) von ungleich null von der Frucht (1) bereitgestellt zu sein, wobei der zweite Messabstand (d2) zwischen null und der Fraunhofer-Distanz der zweiten Mikrowellenantenne (7) zu der Frucht (1) liegt,
- Emittieren eines zweiten Mikrowellensignals (a2) im Mikrowellenfrequenzbereich zur Frucht (1) hin, wenn die zweite Antenne (7) im zweiten Messabstand (d2) von der Frucht (1) bereitgestellt ist; und
- Empfangen eines zweiten reflektierten Mikrowellensignals (b2), wenn die zweite Antenne (7) in dem zweiten Messabstand (d2) von der Frucht (1) bereitgestellt ist, wobei das zweite reflektierte Mikrowellensignal (b2) aus dem zweiten emittierten Mikrowellensignal (a2) resultiert, das von der Frucht (1) in Richtung der zweiten Antenne (7) reflektiert wird,
wobei die Mikrowellenquelle (4) oder eine zweite Mikrowellenquelle mit der zweiten Mikrowellenantenne (7) zum Erzeugen des zweiten emittierten Mikrowellensignals (a2) verbunden ist, und wobei der Prozessor (5) mit der zweiten Mikrowellenantenne (7) verbunden ist, und wobei das Regressionsmodell dazu konfiguriert ist, einen zweiten komplexen Reflexionskoeffizienten der Frucht (1) direkt mit dem einen oder den mehreren Qualitätsattributen der Frucht (1) zu korrelieren,
und wobei die Programmierung auf dem Prozessor (5) ferner zu Folgendem konfiguriert ist:
- direktes Bestimmen, unter Verwendung des Regressionsmodells mit dem bestimmten komplexen Reflexionskoeffizienten und einem zweiten komplexen Reflexionskoeffizienten über den Mikrowellenfrequenzbereich als Eingabe, des einen oder der mehreren Qualitätsattribute der Frucht (1), wobei der zweite komplexe Reflexionskoeffizient über den Mikrowellenfrequenzbereich durch Vergleichen des zweiten emittierten Mikrowellensignals (a2) mit dem zweiten reflektierten Mikrowellensignal (b2) bestimmt wird; und
- Bestimmen der internen Qualität der Frucht (1) basierend auf den Qualitätsattributen.

14. Messsystem nach Anspruch 13, wobei der Prozessor (5) ferner konfiguriert ist zum:
- Bestimmen eines komplexen Transmissionskoeffizienten der Frucht (1) über den Mikrowellenfrequenzbereich durch Vergleichen des ersten emittierten Mikrowellensignals (a1) mit einem transmittierten Mikrowellensignal, das durch die zweite Mikrowellenantenne (7) gemessen wird; und
- Verwenden des Transmissionskoeffizienten als Eingabe für das Regressionsmodell.

## Revendications

1. Procédé de détermination d'une qualité interne d'un fruit (1), dans lequel on utilise un système de mesure par micro-ondes (2) comprenant :
- une antenne micro-ondes (3) pour émettre et recevoir des signaux micro-ondes dans une gamme de fréquences micro-ondes, l'antenne micro-ondes (3) étant configurée pour :
∘ être disposée à une distance de mesure (d1) non nulle d'une surface du fruit (1), la distance de mesure (d1) étant comprise entre zéro et la distance de Fraunhofer de l'antenne micro-ondes (3) par rapport au fruit (1),
∘ émettre un signal micro-onde (a1) dans la gamme de fréquences micro-ondes vers le fruit (1) lorsque l'antenne (3) est disposée à la distance de mesure (d1) de la surface du fruit (1) ; et
∘ recevoir un signal micro-onde réfléchi (b1) lorsque l'antenne (3) est placée à la distance de mesure (d1) du fruit, le signal micro-onde réfléchi (b1) résultant du signal micro-onde émis (a1) réfléchi par le fruit (1) vers l'antenne (3),
- une source de micro-ondes (4) connectée à l'antenne micro-ondes (3) pour générer le signal micro-onde (a1) émis dans la gamme de fréquences micro-ondes ; et
- un processeur (5) connecté à l'antenne micro-ondes (3), et de préférence à la source de micro-ondes (4), le processeur (5) comprenant une programmation exécutant un modèle de régression, de préférence déterminé par une procédure d'étalonnage, configurée pour corréler directement un coefficient de réflexion complexe du fruit (1) avec un ou plusieurs attributs de qualité du fruit (1),
le procédé comprenant :
- la mise en place (101) de l'antenne micro-ondes (3) à la distance de mesure (d1) de la surface du fruit (1) ;
- l'émission (102) du signal micro-onde (a1) dans la gamme de fréquences micro-ondes de l'antenne micro-ondes (3) vers le fruit (1) ;
- la réception (103) du signal micro-onde réfléchi (b1) avec l'antenne (3) ;
- la détermination (104) du coefficient de réflexion complexe du fruit (1) sur la gamme de fréquences micro-ondes en comparant le signal micro-onde émis (a1) au signal micro-onde réfléchi (b1) ;
- la détermination directe (105), par la programmation sur le processeur (5) en utilisant le modèle de régression avec, en tant qu'entrée, le coefficient de réflexion complexe déterminé sur la gamme de fréquences micro-ondes, du ou des attributs de qualité du fruit (1) ;
- la détermination (106), par la programmation sur le processeur (5), de la qualité interne du fruit (1) sur la base des attributs de qualité, en comparant les attributs de qualité à une ou plusieurs valeurs de seuil et en déterminant la qualité interne selon que les attributs de qualité sont supérieurs ou inférieurs à la ou aux valeurs de seuil ; et
- la délivrance (107) par le processeur (5) de la qualité interne déterminée du fruit (1).

2. Procédé selon la revendication 1, dans lequel le système de mesure par micro-ondes (2) comprend en outre une couche non conductrice (6) prévue sur l'antenne micro-ondes (3), la couche non conductrice (6) présentant une épaisseur inférieure à la distance de Fraunhofer de l'antenne micro-ondes (3) à travers la couche non conductrice jusqu'au fruit (1), et le procédé comprenant :
- la mise en place de la couche non conductrice (6) sur la surface du fruit (1) de telle sorte que la couche non conductrice (6) touche le fruit (1) et que l'antenne (3) soit à la distance de mesure (d1) inférieure à la distance de Fraunhofer à travers la couche non conductrice (6) par rapport au fruit (1).

3. Procédé selon une ou plusieurs des revendications précédentes, le procédé comprenant en outre :
- la réalisation (108) d'une transformation dans le domaine temporel sur le coefficient de réflexion complexe ou les micro-ondes émises (a1) et réfléchies (b1) afin de déterminer la distance de mesure (d1) entre le fruit (1) et l'antenne micro-ondes (3) ; et
- l'utilisation de la distance de mesure (d1) déterminée en tant qu'entrée dans le modèle de régression pour déterminer les attributs de qualité.

4. Procédé selon une ou plusieurs des revendications précédentes, le procédé étant utilisé en combinaison avec une bande transporteuse qui est configurée pour transporter le fruit (1), le procédé comprenant en outre :
- le transport du fruit (1) sur la bande transporteuse tout en émettant le signal micro-onde (a1) et en recevant le signal micro-onde réfléchi (b1).

5. Procédé selon une ou plusieurs des revendications précédentes, le procédé comprenant en outre :
- le tri du fruit (1) sur la base de la qualité interne du fruit (1), par exemple en prévoyant un seuil de qualité interne et en triant le fruit (1) selon que la qualité interne du fruit (1) est supérieure ou inférieure au seuil de qualité interne.

6. Procédé selon l'une ou plusieurs des revendications précédentes, dans lequel le système de mesure par micro-ondes (2) comprend une seconde antenne micro-ondes (7) pour émettre et recevoir des signaux micro-ondes dans une gamme de fréquences micro-ondes, la seconde antenne micro-ondes (7) étant configurée pour :
- être disposée à une seconde distance de mesure (d2) non nulle du fruit (1), la seconde distance de mesure (d2) étant comprise entre zéro et la distance de Fraunhofer de la seconde antenne micro-ondes (7) par rapport au fruit,
- émettre un second signal micro-onde (a2) dans la gamme de fréquences micro-ondes vers le fruit (1) lorsque la seconde antenne (7) est disposée à la seconde distance de mesure (d2) du fruit (1) ; et
- recevoir un second signal micro-onde réfléchi (b2) lorsque la seconde antenne (7) est placée à la seconde distance de mesure (d2) du fruit (1), dans lequel le second signal micro-onde réfléchi (b2) résulte du second signal micro-onde émis (a2) réfléchi par le fruit (1) vers la seconde antenne (7),
dans lequel la première source de micro-ondes (4) ou une seconde source de micro-ondes est connectée à la seconde antenne micro-ondes (7) pour générer le second signal micro-onde émis (a2) et dans lequel le processeur (5) est connecté à la seconde antenne micro-ondes (7) et dans lequel le modèle de régression est configuré pour corréler directement un second coefficient de réflexion complexe du fruit (1) avec le ou les attributs de qualité du fruit (1),
et le procédé comprenant en outre :
- la mise en place de la seconde antenne micro-ondes (7) à la seconde distance de mesure (d2) du fruit (1) ;
- l'émission du second signal micro-onde (a2) dans la gamme de fréquences micro-ondes de la seconde antenne micro-ondes (7) vers le fruit (1) ;
- la réception du second signal micro-onde réfléchi (b2) avec la seconde antenne (7) ;
- la détermination du second coefficient de réflexion complexe du fruit (1) sur la gamme de fréquences micro-ondes en comparant le second signal micro-onde émis (a2) au second signal micro-onde réfléchi (b2) ;
- la détermination directe, par la programmation sur le processeur (5) en utilisant le modèle de régression avec, en tant qu'entrée, les premier et second coefficients de réflexion complexes déterminés sur la gamme de fréquences micro-ondes, du ou des attributs de qualité du fruit (1) ; et
- la détermination, par la programmation sur le processeur, de la qualité interne du fruit (1) sur la base des attributs de qualité.

7. Procédé selon la revendication 6, le procédé comprenant en outre :
- la réception par la seconde antenne (7) d'un signal micro-onde transmis provenant de la première antenne (3), le signal micro-onde transmis résultant du premier signal micro-onde transmis par le fruit (1) ;
- la détermination par le processeur (5) d'un coefficient de transmission complexe du fruit (1) sur la gamme de fréquences micro-ondes en comparant le premier signal micro-onde émis au signal micro-onde transmis ; et
- l'utilisation du coefficient de transmission en tant qu'entrée dans le modèle de régression.

8. Système de mesure par micro-ondes (2) pour déterminer une qualité interne d'un fruit (1), comprenant :
- une antenne micro-ondes (3) pour émettre et recevoir des signaux micro-ondes dans une gamme de fréquences micro-ondes, l'antenne micro-ondes (3) étant configurée pour :
∘ être disposée à une distance de mesure non nulle (d1) du fruit (1), la distance de mesure (d1) étant comprise entre zéro et la distance de Fraunhofer de l'antenne micro-ondes (3) par rapport au fruit (1),
∘ émettre un signal micro-onde (a1) dans la gamme de fréquences micro-ondes vers le fruit (1) lorsque l'antenne (3) est disposée à la distance de mesure (d1) du fruit (1) ; et
∘ recevoir un signal micro-onde réfléchi (b1) lorsque l'antenne (3) est placée à la distance de mesure (d1) du fruit (1), le signal micro-onde réfléchi (b1) résultant du signal micro-onde émis (a1) réfléchi par le fruit (1) vers l'antenne (3),
- une source de micro-ondes (4) connectée à l'antenne micro-ondes (3) pour générer le signal micro-onde (a1) émis dans la gamme de fréquences micro-ondes ; et
- un processeur (5) connecté à l'antenne micro-ondes (3), et de préférence à la source de micro-ondes (4), le processeur (5) comprenant une programmation exécutant un modèle de régression, de préférence déterminé par une procédure d'étalonnage, configurée pour corréler directement un coefficient de réflexion complexe du fruit (1) avec un ou plusieurs attributs de qualité du fruit (1),
dans lequel la programmation sur le processeur (5) est configurée pour :
- déterminer directement, en utilisant le modèle de régression avec, en tant qu'entrée, un coefficient de réflexion complexe sur la gamme de fréquences micro-ondes, le ou les attributs de qualité du fruit (1), dans lequel le coefficient de réflexion complexe est déterminé sur la gamme de fréquences micro-ondes en comparant le signal micro-onde émis (a1) au signal micro-onde réfléchi (b1) ;
- déterminer la qualité interne du fruit (1) sur la base des attributs de qualité, en comparant les attributs de qualité à une ou plusieurs valeurs de seuil et fournir une étiquette de qualité selon que les attributs de qualité sont supérieurs ou inférieurs à la ou aux valeurs de seuil; et
- délivrer la qualité interne déterminée du fruit (1).

9. Système de mesure selon la revendication 8, le système de mesure par micro-ondes (2) comprenant en outre une couche non conductrice (6) prévue sur l'antenne micro-ondes (3), la couche non conductrice (6) présentant une épaisseur inférieure à la distance de Fraunhofer de l'antenne micro-ondes (3) à travers la couche non conductrice (6) jusqu'au fruit (1), et l'antenne (3) étant configurée pour être placée avec la couche non conductrice (6) sur le fruit (1) de telle sorte que la couche non conductrice (6) touche le fruit (1) et que l'antenne (3) soit à la distance de mesure (d1) inférieure à la distance de Fraunhofer à travers la couche non conductrice par rapport au fruit (1).

10. Système de mesure selon l'une ou plusieurs des revendications 8 à 9, dans lequel le processeur (5) est en outre configuré pour :
- réaliser une transformation dans le domaine temporel sur le coefficient de réflexion complexe ou sur les micro-ondes émises (a1) et réfléchies (b1) afin de déterminer la distance de mesure (d1) entre le fruit (1) et l'antenne micro-ondes (3) ; et
- utiliser la distance de mesure (d1) en tant qu'entrée dans le modèle de régression.

11. Système de mesure selon une ou plusieurs des revendications 8 à 10, dans lequel le système de mesure (2) comprend en outre une bande transporteuse qui est configurée pour transporter le fruit (1).

12. Système de mesure selon une ou plusieurs des revendications 8 à 11, dans lequel le processeur (5) est en outre configuré pour trier le fruit (1) sur la base de la qualité interne du fruit (1), par exemple en triant le fruit (1) selon que la qualité interne du fruit (1) est supérieure ou inférieure à un seuil de qualité interne.

13. Système de mesure selon une ou plusieurs des revendications 8 à 12, dans lequel le système de mesure par micro-ondes (2) comprend une seconde antenne micro-ondes (7) pour émettre et recevoir des signaux micro-ondes dans une gamme de fréquences micro-ondes, la seconde antenne micro-ondes (7) étant configurée pour :
- être disposée à une seconde distance de mesure (d2) non nulle du fruit (1), la seconde distance de mesure (d2) étant comprise entre zéro et la distance de Fraunhofer de la seconde antenne micro-ondes (7) par rapport au fruit (1),
- émettre un second signal micro-onde (a2) dans la gamme de fréquences micro-ondes vers le fruit (1) lorsque la seconde antenne (7) est disposée à la seconde distance de mesure (d2) du fruit (1) ; et
- recevoir un second signal micro-onde réfléchi (b2) lorsque la seconde antenne (7) est disposée à la seconde distance de mesure (d2) du fruit (1), le second signal micro-onde réfléchi (b2) résultant du second signal micro-onde émis (a2) réfléchi par le fruit (1) vers la seconde antenne (7),
dans lequel la source de micro-ondes (4) ou une seconde source de micro-ondes est connectée à la seconde antenne micro-ondes (7) pour générer le second signal micro-onde émis (a2) et dans lequel le processeur (5) est connecté à la seconde antenne micro-ondes (7) et dans lequel le modèle de régression est configuré pour corréler directement un second coefficient de réflexion complexe du fruit (1) avec le ou les attributs de qualité du fruit (1), et dans lequel la programmation sur le processeur (5) est en outre configurée pour :
- déterminer directement, en utilisant le modèle de régression avec, en tant qu'entrée, le coefficient de réflexion complexe déterminé et un second coefficient de réflexion complexe sur la gamme de fréquences micro-ondes, le ou les attributs de qualité du fruit (1), le second coefficient de réflexion complexe étant déterminé sur la gamme de fréquences micro-ondes en comparant le second signal micro-onde émis (a2) au second signal micro-onde réfléchi (b2) ; et
- déterminer la qualité interne du fruit (1) sur la base des attributs de qualité.

14. Système de mesure selon la revendication 13, dans lequel le processeur (5) est en outre configuré pour :
- déterminer un coefficient de transmission complexe du fruit (1) sur la gamme de fréquences micro-ondes en comparant le premier signal micro-onde émis (a1) à un signal micro-onde transmis mesuré par la seconde antenne micro-ondes (7) ; et
- utiliser le coefficient de transmission en tant qu'entrée pour le modèle de régression.
